# EUROPEAN PATENT SPECIFICATION

(11) **EP 1 935 448 B1**
(45) Date of publication and mention of the grant of the patent: **05.06.2013**
(21) Application number: 07024523.8
(22) Date of filing: 18.12.2007
(51) Int. Cl.: A61N 1/05

(54) **Implantable medical lead having coil electrode**
Implantierbare medizinische Elektrodenleitung mit Spulenelektrode
Câble médical implantable doté d'une électrode en spirale

(30) Priority: 18.12.2006 US 870531 P; 14.12.2007 US 956724
(43) Date of publication of application: 25.06.2008
(73) Proprietor: Greatbatch Ltd., Clarence, NY 14031 (US)
(72) Inventor: Zweber, Jeffery, St. Louis Park, MN 55426 (US); Geroy, Jesse, North St. Paul, MN 55109 (US); Ye, Qingshan, Plymouth, MN 55441 (US)
(74) Representative: Koepe, Gerd L.

(56) References cited:
- EP-A- 0 442 444
- US-A- 4 161 952
- US-A- 4 817 634
- US-A- 4 922 607
- US-A- 4 934 049
- US-A- 6 052 625
- US-A1- 2006 241 734

## Description

### BACKGROUND OF THE INVENTION

### 1. Field of the Invention

The present invention is related to implantable electrical medical stimulation and sensing leads. More specifically, the present invention is related to implantable leads with a coil electrode having the coil inter-turn space filled with polymer.

### 2. Prior Art

Implantable electrical leads are well known. Such leads are used in many sensing and stimulation applications. Cardiac leads are used for both sensing and stimulation, for recording, pacing, and defibrillation. Some cardiac leads are used in bi-ventricular pacing for cardiac resynchronization therapy by advancing the lead through the cardiac sinus and into a cardiac vein to pace the left ventricle. Neurological leads have been used for spinal stimulation and for deep brain stimulation (DBS) applications. Other applications include sensing and stimulation for gastric applications.

Neurological leads often require a larger number of conductors compared to cardiac applications. Some pacing applications only require one or two conductors, while some neurological applications may utilize eight distinct conductors. The leads for many neurological applications require smaller and more flexible leads than for many previous cardiac type applications. In one application, a stimulator is implanted in the front of the abdomen, and a lead or lead extension is tunneled around to the back, under the skin. The lead having multiple distal conductors is often inserted into the epidural space and advanced along the spinal cord, until the multiple electrodes are properly positioned. Such electrodes are typically cylindrical ring electrodes coupled through conductors to ring connector contacts at the proximal end. A small outer diameter and good flexibility is required to achieve the optimal result.

Peripheral nerve stimulation has become more common, and would often benefit from even smaller lead sizes and greater flexibility. It may be desirable to run leads under the skin along the neck, and along at least part of the head or face. It may also be desirable to run leads under the skin along side joints and other parts of the body that may flex, stretch, and/or rotate.

Neurological leads often have distal band electrodes having a cylindrical shape. Such band electrodes are typically formed of thin metal bands. Such bands are typically secured on the surface of a polymeric tube, and coupled to thin conductors. The tube often has a lumen, occupied by a stiffening member or stylet during delivery. The stylet can be inserted to provide column strength to allow for pushing the lead through a needle and to the target site. The stylet can then be removed. Without the stylet, the lead is often rather limp, and could not easily be advanced without the added stiffness. Leads implanted under the skin and having distal band electrodes can rub against the skin. Patients having such implanted leads may be acutely aware of any inflexible lead presence over time.

The document EP-A 442 444 discloses an implantable electrode of the type in which an elongated wire coil forms the electrode surface. The electrode coil is mounted around an insulative lead body and is stabilized on the lead body by means of a plastic filler between the individual turns of the electrode coil, extending radially outward to approximately one-third of the diameter of the electrode coil wire. The filler is produced by stretching a portion of the lead body which normally displays an outer diameter greater than the inner diameter of the electrode coil, sliding the electrode coil over the lead body and inserting a mandral into the lead body to urge the lead body into contact with the electrode coil. This assembly is heated to encourage flow of the lead body into the spaces between the electrode coil to stabilize the coil on electrode body and to prevent fibrotic ingrowth around the electrode coil wire.

The document US-A 200610,241,734 relates to a medical electrical electrode includes an elongated conductive coil located over a lead body, and a conductive polymer material in contact with the lead body and located between individual coils of the elongated conductive coil. In certain embodiments, the conductive polymer is a polymer (e.g., silicone) implanted with a conductive filler (e.g., carbon black). In certain embodiments, the conductive polymer material is generally isodiametric with an outer diameter of the individual coils of the elongated conductive coil.; A medical electrical electrode is fabricated by sliding an elongated conductive coil over a length of a lead body, dispersing a conductive polymer on the helical coil, inserting a tubing over the elongated conductive coil, distributing the polymer material between individual turns of the elongated conductive coil, heating the tubing so the tubing shrinks around the elongated conductive coil, and removing the tubing.

What would be desirable are implantable leads having smaller outer diameters and more flexible distal regions.

### SUMMARY OF THE INVENTION

The invention relates to an implantable medical electrical lead according to claim 1.

The present invention provides an implantable medical electrical lead including a flexible elongate tubular body having a proximal region and a distal region, and having a first coil electrode wound around at least a portion of the lead distal region. The coil includes a plurality of coil turns, a distance between the coil turns, and an outer surface to the coil. The first coil has a polymeric material disposed between the turns, with the first coil outer surface being substantially free of the polymeric material, such that the first coil outer surface is electrically conductive with a surrounding environment.

The tubular body has a lumen disposed through at least part of the lead length. Leads may have an outside diameter of less than about 1,27 or 0,899 mm (0.050 or 0.035 inch), in various embodiments. Some leads are formed of polyurethane or silicone rubber. Leads include a first conductor extending from the proximal region to the distal region, the first conductor being in electrical continuity with the first coil electrode wherein the first conductor is integrally formed with the first coil electrode and extends through the lower.

Disclosed, but not covered by the claims, are methods for making an implantable medical lead. One method includes aligning a plurality of electrically conductive coils along a longitudinal axis, wherein the coils include a plurality of conductor turns, an outer surface, and a space between the conductor turns. The method also includes masking the coils' outer surface with a masking material and infusing a polymer or polymeric precursor material in between the coil turns, but substantially not covering the outer surface of the coils. The polymer or polymeric precursor is allowed to become more solid, such that a polymeric material extends between the coil turns, followed by removing the masking material, such that an electrically conductive coil outer surface is exposed.

Disclosed, but not covered by the claims, are methods for pre-loading the polymer or polymeric precursors with limited amount of drugs, such as dexamethasone sodium phosphate, or modifying/grafting the polymer precursors or polymer itself with functional small or large molecules, and infusing the drug-loaded or modified polymer/polymer precursors between the coils' turns.

In some methods, the coils are disposed over a mandrel during the aligning, the method further comprising removing the mandrel at some point after the solidifying, leaving a lumen though the lead body. In other methods the coils are disposed over a solid polymeric shaft during the aligning, where the solid polymeric shaft is not removed. In still other methods, the coils are disposed over a tubular polymeric shaft having a lumen there through during the aligning, where the tubular polymeric shaft is not removed.

The masking material is a shrinkable sheath disposed over the coils in some methods, in which the masking includes shrinking the shrinkable sheath. The sheath may be heat shrinkable in some methods. The masking can include urging a

mold inner surface against the coils, and/or disposing the coils within a mold.

Disclosed, but not covered by the claims, are methods for implanting neurological electrical leads. One method includes advancing a hollow delivery needle through the skin, and advancing an electrical lead through the needle, where the lead has a plurality of distal electrodes formed of electrically conductive coils having a substantially electrically conductive outer surface and a substantially filled space inbetween the coil turns. The advancing can be stopped when the lead is disposed near a target site, followed by withdrawing the delivery needle. In some methods, the lead has a lumen there through, and the method further includes disposing a stiffening member through the lead prior to the advancing, and removing the stiffening member. Some methods include advancing a delivery sheath through the needle, in which at least some of the lead advancing is performed through the advanced delivery sheath and/or advanced together with the advancing delivery sheath at the same time.

The sheath has a distal region which is steerable in some methods. The lead advancing can include advancing through the spine epidural space, in which sheath distal region is steered to direct the lead toward a nerve root, further comprising advancing the lead to the nerve root. The lead can be advanced toward a peripheral nerve site in some methods. Mapping can be performed in some methods using the lead distal electrodes, through holes or slots in the sheath distal region. Separate mapping electrodes on the sheath distal region may be used in other methods.

### BRIEF DESCRIPTION OF THE DRAWINGS

FIG. 1 is a fragmentary, perspective view of a lead according to the present invention having distal coil electrodes and proximal cylindrical ring connector contacts.
FIG. 2 is fragmentary view of the lead of FIG. 1, showing the distal region with coil electrodes and a distal portion of a stylet adapted to slidably fit within the lead to stiffen the lead.
FIG. 3 is a fragmentary view of the lead of FIG. 1, showing the proximal region used to electrically couple to an electrical medical device or a lead extension.
FIG. 4 is a close up, fragmentary view of the distal region of the lead of FIG. 1, showing the coil electrodes and conductors connected to the coils.
FIG. 5A is a transverse, cross-sectional view of a lead body having a lumen within for receiving a stiffening member.
FIG. 5B is a transverse, cross-sectional view of a lead body not having a lumen within.
FIGs. 6A, 6B and 6C show one example of use of the lead of Fig. 1 in a method for introducing the lead of FIG 1, through a steerable sheath introduced through a needle, which is not covered by the claims.

### DETAILED DESCRIPTION OF THE PREFERRED EMBODIMENTS

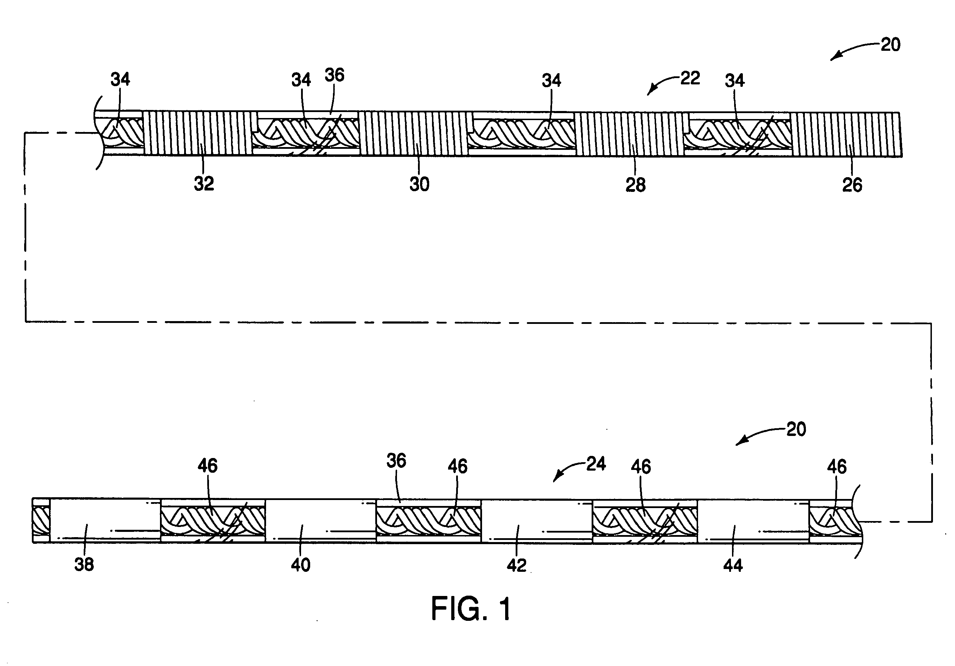

FIG. 1 illustrates a lead 20 according to the present invention having a distal region 22 and a proximal region 24. Distal region 22 includes several coil electrodes 26, 28, 30, and 32 separated by tubular separating or inter-coil regions 34. Proximal region 24 includes several connector contacts 38, 40, 42, and 44, separated by tubular separating regions 46. The connector contacts may be formed of a metal such as Nitinol, MP35N, platinum/iridium, stainless steel, titanium, or other metals typically used in the medical device arts. The connector contacts are typically cylindrical or band shaped, formed of a cylinder of metal wrapped around the lead body, rather than being formed of a coil. Proximal region 24 is often inserted into an electrical medical device, for example, an implantable monitor, or an implantable neurostimulator or the like. Proximal region 24 can also be inserted into a lead extension device to extend the effective length between the lead and the electrical medical device.

The coil electrodes, such as coil electrode 26, can be formed of metals, for example, MP35N, platinum/iridium, stainless steel, titanium, and gold, and may have an outer diameter of between about 0.254 mm (0.010 inch) and 1.27 mm (0.050 inch), likely having a size less than about 0.899 mm (0.035 inch). The coils have between about 3 and 100 turns per coil in some embodiments, usually at least three turns. In some embodiments, the OD of the coil is less than about 0.889, 0.762, 0.7112, 0.635, or 0.508 mm (0.035, 0.030, 0.028, 0.025, or 0.020 inches). The lead body can be formed of polyurethane or a silicone rubber in some embodiments.

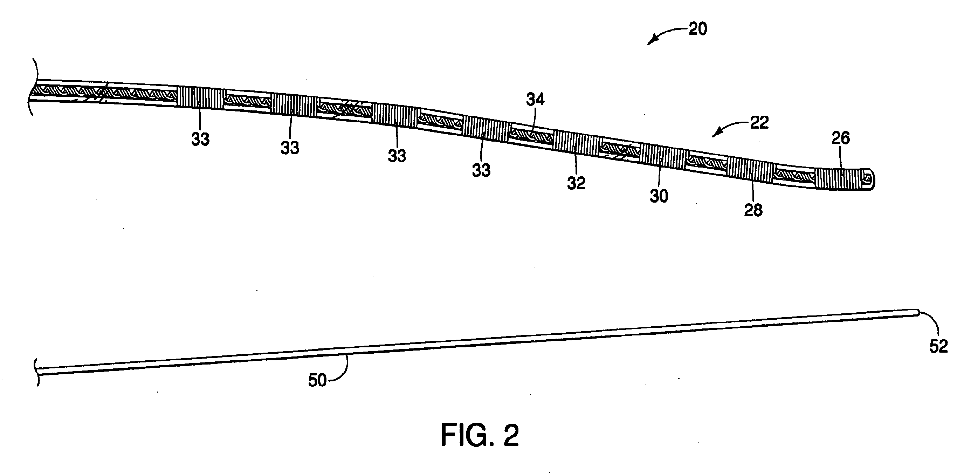

FIG. 2 illustrates a prototype of lead 20, in distal region 22. Coil electrodes 26, 28, 30, and 32 are shown as previously discussed, along with four more coil electrodes all numbered as 33. Inspection of FIG. 2 shows that the lead distal region 22 can flex and stretch more than a similar lead having solid band electrodes. Close inspection of inter-coil regions 34 may reveal the multi-strand conductors which run through the lead and are coupled to the coil electrodes. Such conductors typically are individually coupled, one each to a coil electrode, such that the coil electrodes are individually electrically coupled to the proximal end. While not readily visible from FIG. 2, the coil electrodes have polymer disposed between the coil turns, but not covering at least a portion of the outer surface of the coils. This makes the coils function as conductive electrodes, while providing some added strength to the lead and inhibiting later in-growth of biological material.

The distance between each turn is from about 2.54 µm (0.0001 inches) to about 2.54 mm (0.1 inches) axially along the longitudinal axis of the lead 20, and this space is occupied by the polymeric material 36. The polymeric material 36 also forms the lead outer surface between the respective coil electrodes in the inter-coil regions 34.

A stylet 50 is also shown in FIG. 2, including a distal end 52. Stylet 50 can be slidably inserted within a lumen in lead 20, to stiffen the lead during the lead advance to the target site. As is discussed below, some leads do not require stylets or stylet lumens.

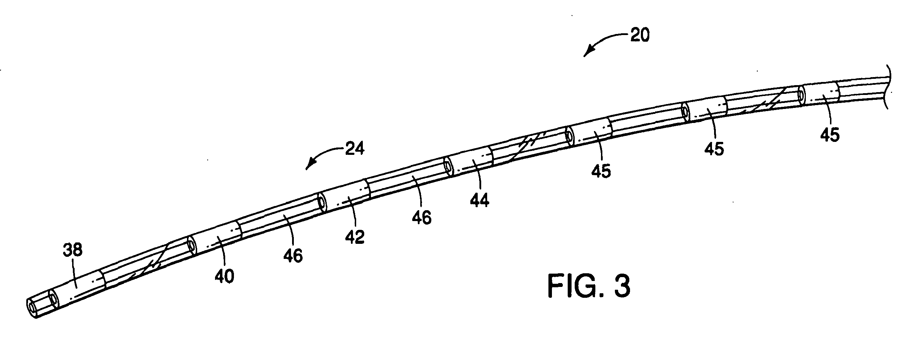

FIG. 3 illustrates lead 20 proximal region 24, having connector contacts 38, 40, 42, and 44, as previously discussed, along with four more contacts all numbered 45. Inter-electrode regions 46 may also be seen. The contacts are typically individually electrically coupled, each to a distal coil electrode. Inspection of FIG. 3 shows that lead proximal region 24 is not as flexible in going around a tight bend, relative to distal region 22 having the coils. In addition, the solid bands will not stretch as will the coil electrodes. Proximal region 24 may have a port to admit a stylet in some embodiments, while other embodiments have such a stylet entry port in the mid region, while others have no stylet lumen.

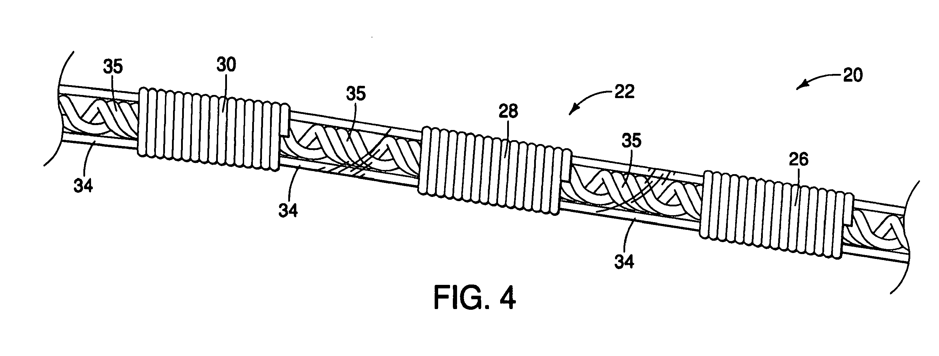

FIG. 4 shows lead distal region 22 in more detail. Multiconductors 35 may be seen extending between the coil electrodes 26, 28, and 30 in inter-coil regions 34. The coil electrodes are seen to have about 12 turns in this prototype.

Both coil and conductor may be originally formed of a contiguous electrical conductor. The conductor coupled to each coil may be formed of a single wire for each unique channel in some embodiments and of multi-strand wires in other embodiments. The multi-strand wires may have different configurations in different embodiments, including small diameter coils, which may increase in coil diameter to form the coil electrodes in some embodiments. As previously discussed, the coil turns can have polymer filling the region between the turns, while not covering the outer extent of the coil. Some polymer filling includes a silicone-polyurethane blend.

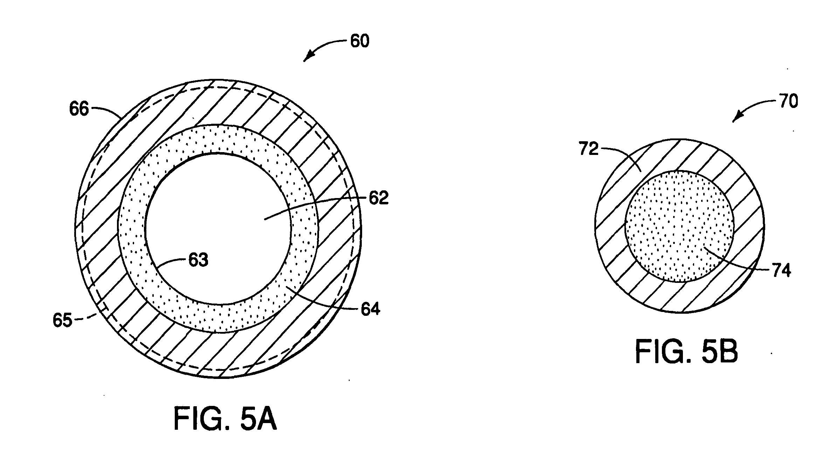

FIG. 5A illustrates one lead in a quasi-transverse cross-section cutting through a distal coil 66, which has an inner polymer layer 64 having a lumen 62 within. Polymer layer 64 extends from an inner wall 63 to an outer extent 65, shown in projection behind coil 66. The conductors may be disposed within polymer layer 64 in some embodiments. A multiconductor wire cable may have the individual conductors arranged side-by-side in a somewhat flattened cable, with the cable itself spirally arranged around the outside of lumen 62, for example, within polymer layer 64. FIG. 5A actually is a somewhat helically slanted view, through a coil turn, as a strictly transverse slice would show at least some of the polymer between the coil turns, as the coil turn advanced. A helical view through an inter-turn region would likely show polymer where coil 66 is located in FIG. 5A. This added thickness of polymer may be seen to add to the lead wall thickness and to the column strength of the lead, relative to a lead having empty space strength of the lead, relative to a lead having empty space between the coil turns. Just as the coil may, in some embodiments, be formed of a single wire strand that coils, the inter-strand region may (absent manufacturing irregularities) be formed of a single strand of polymer that also coils, alternating with the wire strands.

FIG. 5B illustrates another lead 70 in a quasi-transverse cross-section through one coil strand 72. Lead 70 does not have a lumen, but has a solid polymer center 74, with the polymer extending outward to fill in the inter-strand regions with polymer. The added strength of the polymer filled coils can allow a decrease in lead outer diameter, and even eliminate the need for a stiffening member in some embodiments.

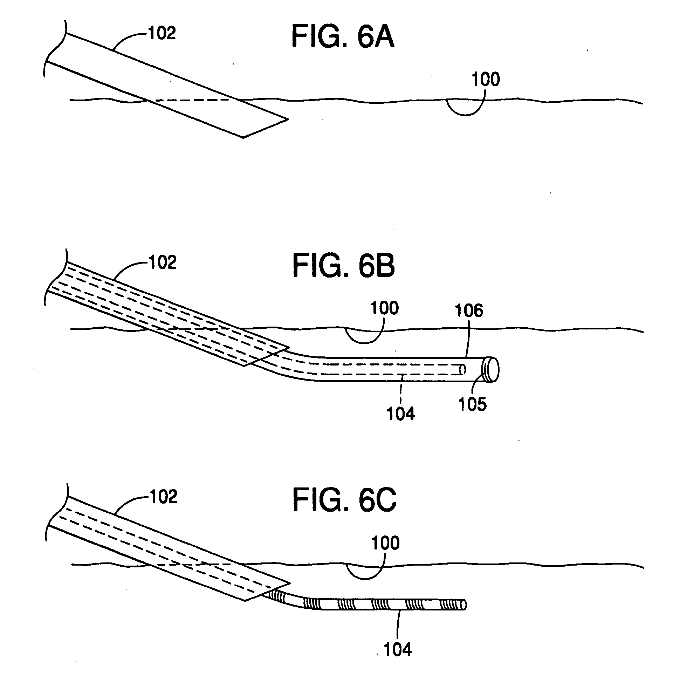

FIGs. 6A to 6C show one lead in one example of use. A needle 102 is inserted under skin 100, and a steerable delivery catheter or sheath 106 advanced through needle 102. Steerable delivery sheaths are well known in the art.

FIG. 6B shows a lead 104 advanced through the sheath 106. The steerable sheath, either alone, or having a lead within, may be sufficiently strong and stiff to advance through a difficult, even scarred tissue path. A stylet may not be required. A distal mapping electrode 105 may be included on sheath 106 and electrically coupled to a sheath proximal region (not shown in FIG. 6B). The mapping electrode may be used to determine the proper location for placing the lead, even before advancing the lead out of the sheath.

FIG. 6C illustrates lead 104 after the sheath has been withdrawn, leaving lead 104 in place.

In some applications, lead 70 may be inserted by itself into a human or mammalian body and advanced to the target site without use of a sheath, introducer, or needle. In some applications, the lead is inserted alone or having only a stiffener. In other applications, an introducer or sheath is used, without a needle. In some coronary applications, the lead may be advanced to the target site (e.g. the coronary sinus or a cardiac vein) using conventional methods used for reaching those target sites. In some coronary applications, the various coils may be individually addressed during surgery or long after surgery, in order to determine the proper coil combination for obtaining the optimal clinical result. In one such example, the various coils may be stimulated one by one or in various combinations to determine the best coil or coils to use for LV pacing from a cardiac vein.

In some applications, the sheath has its own set of one or more external distal electrodes which can be used in mapping, to locate the optimal site for placement of the lead. In other applications, the sheath has distal holes or slots through the sheath side wall to allow the lead distal electrodes to electrically contact the surrounding tissue. The lead itself can be used for mapping purposes while still disposed in the partially surrounding sheath.

Devices according to the present invention can be made using various methods. The manufacture of polymeric leads and catheters is well known to those skilled in the art, with millions made each year. The details of the already known aspects of those methods are well documented in the patent literature of the past decades. These details need not be repeated here. Methods for allowing molten polymers to solidify, and for pre-polymers to react and cure (e.g. monomers and catalysts) in place are also well known.

One lead, having a lumen according to the present invention can be made using various methods. A removable solid mandrel shaft or a removable tube can be used to hold a series of distal coil electrodes, conductors, and proximal connector contacts, in their ultimate position around and along the shaft or tube. A shrink wrap or heat shrink material can be placed over the subassembly and shrunk into place using heat application or other methods. The shrunken material can be used to cover the outer extent of the coils and connector contacts, to mask them from later added polymer. Preferably, the heat shrink material covers an area extending from about 10° to about 180° around the outwardly facing circumference of each coil turn. This is the same area that will eventually be devoid of polymeric material after the heat shrink material is removed. In some methods, the shrink wrap material is either not applied in between the coil electrodes or is applied, then removed prior to the polymer application.

The device can be placed in a tubular mold, for example, two opposing concave half-tubes which are brought together about the tubular device. A flowable polymeric material can then be infused into the mold to the masked coil electrodes and the remainder of the device. This flowable polymeric material can include a heated thermoplastic material or a pre-polymeric material including monomer. After the polymer has solidified or cured, the mold can be removed. The masking material can be removed to expose the still conductive outer surfaces of the coil electrodes comprising the area extending from about 10° to about 180° around the outwardly facing circumference of each coil turn that was previously covered by the heat shrink material. Most or all of the coil inter-turn regions may be filled with the polymer. The presence of this polymer rather than empty space increases the effective wall thickness and strength of the lead.

In another method, a lead not having a lumen can be created. The coil electrodes, conductors, and connector contacts may be aligned in a mold without the removable mandrel, and the masking applied, followed by applying pourable polymer or pre-polymer solution. After the polymer has solidified, the device can be removed from the mold.

In yet another method, the lead can be formed without a lumen but having a non-removable shaft used to provide some stiffness and/or to properly align the coil electrodes. After masking and application of the polymer, the shaft can be left in the lead body. In still another method, a non-removable tube can be used to align the coils, and later be left in place. This tube lumen may or may not later serve as a stylet lumen.

In some embodiments of the invention, the polymeric material in the coil electrodes may include a medicant formulated by itself or in conjunction with the polymer to elute from the polymer over time. Examples of controlled release formulations, biodegradable polymers, hydrolytically degradable polymers, bioerodable polymers, etc, are well known to those skilled in the art for other uses, such as for coated stents. Some embodiments include one or more steroids in the polymeric material. Some embodiments include glucocorticoid type steroids, for example dexamethasone (the formal name of which is believed to be 9-fluoro-11β,17,21-trihydroxy-16a-methylpregna-1,4-diene-3,20-dione). Such a steroid eluting lead may be particularly beneficial for leads placed in a cardiac vein for LV pacing and/or sensing applications.

Various examples of various embodiments have been described to illustrate some aspects of the invention. The scope of the invention is given in the claims which follow.

## Claims

1. An implantable medical electrical lead (20) having a length, the lead (20) comprising:
a) a flexible elongate body having a proximal region (24) and a distal region (22), wherein the elongate body has at least one lumen disposed through at least part of the lead length;
b) a first coil electrode (26) wound around at least a portion of the lead distal region (22), wherein the first coil electrode (26) has at least one electrically conductive strand having an outer surface and including a plurality of coil turns with a distance between the coil turns;
c) the first coil electrode (26) having a polymeric material (36) disposed between the coil turns;
d) the outer surface of the first coil (26) being substantially free of the polymeric material (36) so that the first coil electrode outer surface is electrically conductive with a surrounding environment;
e) a first conductor extending from the proximal region (24) to the distal region (22), the first conductor being in electrical continuity with the first coil electrode(26); and
f) wherein the first conductor is integrally formed with the first coil electrode (26) and extends through the lumen.

2. The medical lead (20) of claim 1, wherein the outer surface of the first coil (26) being substantially free of the polymeric material (36) extends from 10° to 180° around the outwardly facing circumference of each coil turn.

3. The medical lead (20) of any of claims 1 or 2, wherein the elongate body has an outside diameter of less than 2,54 mm (0.100 inch).

4. The medical lead (20) of any of claims 1 to 3, wherein the first coil electrode (26) has at least three turns.

5. The medical lead (20) of any of claims 1 to 4, wherein the polymeric material (36) includes at least one of polyurethane and silicone.

6. The medical lead (20) of any of claims 1 to 5, wherein the distance between coil turns occupied by the polymeric material (36) is from 2.54 µm to 2.54 mm (0.0001 inches to 0.1 inches) extending axially along a longitudinal axis of the lead (20).

7. The medical lead (20) of any of claims 1 to 6, further comprising at least one additional coil electrode (28, 30, ...) having polymer disposed between the coil turns but not covering at least a portion of an outer surface of the coil electrode.

8. The medical lead (20) of any of claims 1 to 5, wherein at least some of the polymeric material (36) includes a medicant, preferably wherein the medicant includes a time releasable steroid.

## Patentansprüche

1. Implantierbare medizinische Elektrodenleitung (20) mit einer Länge, wobei die Leitung (20) aufweist:
a) einen flexiblen länglichen Körper mit einer proximalen Region (24) und einer distalen Region (22), wobei der längliche Körper mindestens ein Lumen aufweist, das durch mindestens einen Teil der Leitungslänge angeordnet ist;
b) eine erste Spulenelektrode (26), die um mindestens einen Abschnitt von der distalen Region (22) der Leitung gewickelt ist, wobei die erste Spulenelektrode (26) mindestens einen elektrisch leitenden Draht aufweist, der eine Außenoberfläche aufweist und mehrere Spulenwindungen mit einem Abstand zwischen den Spulenwindungen enthält;
c) wobei die erste Spulenelektrode (26) ein Polymermaterial (36) aufweist, das zwischen den Spulenwindungen angeordnet ist;
d) wobei die Außenoberfläche von der ersten Spule (26) im Wesentlichen frei von dem Polymermaterial (36) ist, so dass die Außenoberfläche der ersten Spulenelektrode elektrisch leitend mit einer umliegenden Umgebung ist;
e) einen ersten Leiter, der sich von der proximalen Region (24) zu der distalen Region (22) erstreckt, wobei der erste Leiter mit der ersten Spulenelektrode (26) elektrisch verbunden ist; und
f) wobei der erste Leiter integral mit der ersten Spulenelektrode (26) geformt ist und sich durch das Lumen erstreckt.

2. Medizinische Leitung (20) nach Anspruch 1, wobei die Außenoberfläche von der ersten Spule (26), die im Wesentlichen frei von dem Polymermaterial (36) ist, sich von 10° bis 180° um den nach außen zeigenden Umfang von jeder Spulenwindung erstreckt.

3. Medizinische Leitung (20) nach Anspruch 1 oder 2, wobei der längliche Körper einen Außendurchmesser von weniger als 2.54 mm (0.100 Inch) aufweist.

4. Medizinische Leitung (20) nach einem der Ansprüche 1 bis 3, wobei die erste Spulenelektrode (26) mindestens drei Windungen aufweist.

5. Medizinische Leitung (20) nach einem der Ansprüche 1 bis 4, wobei das Polymermaterial (36) mindestens eines von Polyurethan und Silikon enthält.

6. Medizinische Leitung (20) nach einem der Ansprüche 1 bis 5, wobei die Distanz zwischen Spulenwindungen, die durch das Polymermaterial (36) besetzt ist, von 2.54 µm bis 2.54 mm (0.0001 Inch bis 0.1 Inch) beträgt, die sich axial entlang einer Längsachse von der Leitung (20) erstrecken.

7. Medizinische Leitung (20) nach einem der Ansprüche 1 bis 6, weiter aufweisend mindestens eine zusätzliche Spulenelektrode (28, 30, ...), bei der Polymer zwischen den Spulenwindungen angeordnet ist, aber mindestens ein Teil von einer Außenoberfläche von der Spulenelektrode nicht bedeckt ist.

8. Medizinische Leitung (20) nach einem der Ansprüche 1 bis 5, wobei mindestens etwas von dem Polymermaterial (36) ein Pharmakon enthält, vorzugsweise wobei das Pharmakon ein mit der Zeit abgebbares Steroid enthält.

## Revendications

1. Câble électrique médical implantable (20) ayant une longueur, le câble (20) comprenant :
a) un corps allongé souple ayant une région proximale (24) et une région distale (22), le corps allongé ayant au moins un lumen disposé à travers au moins une partie de la longueur du câble ;
b) une première électrode à bobine (26) enroulée autour d'au moins une partie de la région distale de câble (22), la première électrode à bobine (26) ayant au moins un brin électriquement conducteur ayant une surface externe et comprenant une pluralité de spires de bobine avec une distance entre les spires de bobine ;
c) la première électrode à bobine (26) ayant un matériau polymère (36) disposé entre les spires de bobine ;
d) la surface externe de la première bobine (26) étant essentiellement dépourvue du matériau polymère (36) de sorte que la surface externe de la première électrode à bobine est électriquement conductrice avec un environnement immédiat ;
e) un premier conducteur s'étendant de la région proximale (24) vers la région distale (22), le premier conducteur étant en continuité électrique avec la première électrode à bobine (26) ; et
f) le premier conducteur étant formé d'un seul tenant avec la première électrode à bobine (26) et s'étendant à travers la lumière.

2. Câble médical (20) selon la revendication 1, dans lequel la surface externe de la première bobine (26) qui est essentiellement dépourvue du matériau polymère (36) s'étend de 10° à 180° autour de la circonférence, orientée vers l'extérieur, de chaque spire de bobine.

3. Câble médical (20) selon l'une quelconque des revendications 1 ou 2, dans lequel le corps allongé a un diamètre extérieur inférieur à 2,54 mm (0,100 pouce).

4. Câble médical (20) selon l'une quelconque des revendications 1 à 3, dans lequel la première électrode à bobine (26) a au moins trois spires.

5. Câble médical (20) selon l'une quelconque des revendications 1 à 4, dans lequel le matériau polymère (36) comprend au moins l'un parmi le polyuréthane et la silicone.

6. Câble médical (20) selon l'une quelconque des revendications 1 à 5, dans lequel la distance entre les spires de bobines occupée par le matériau polymère (36) va de 2,54 µm à 2,54 mm (0,0001 pouce à 0,1 pouce) s'étendant axialement le long de l'axe longitudinal du câble (20).

7. Câble médical (20) selon l'une quelconque des revendications 1 à 6, comprenant en outre au moins une électrode à bobine (28, 30, ...) supplémentaire ayant un polymère disposé entre les spires de bobine mais ne couvrant pas au moins une partie d'une surface externe de l'électrode à bobine.

8. Câble médical (20) selon l'une quelconque des revendications 1 à 5, dans lequel au moins une partie du matériau polymère (36) comprend un médicament, de préférence dans lequel le médicament comprend un stéroïde à libération dans le temps.
